# EUROPEAN PATENT SPECIFICATION

(11) **EP 1 931 793 B1**
(45) Date of publication and mention of the grant of the patent: **08.05.2013**
(21) Application number: 06794670.7
(22) Date of filing: 05.10.2006
(51) Int. Cl.: C12P 7/06, C12R 1/01

(54) **METHOD FOR CULTURING MICROORGANISMS**
VERFAHREN ZUR KULTIVIERUNG VON MIKROORGANISMEN
METHODE DE CULTURE DE MICRO-ORGANISMES

(30) Priority: 06.10.2005 GB 0520344
(43) Date of publication of application: 18.06.2008
(73) Proprietor: TMO Renewables Limited, Guildford, Surrey GU2 7YF (GB)
(72) Inventor: ATKINSON, Anthony, Wiltshire SP4 6JL (GB); CRIPPS, Roger, Wiltshire SN16 90T (GB); ELEY, KIRSTIN, Guilford, Surrey GU2 7YF (GB)
(74) Representative: Jappy, John William Graham
(86) International application number: PCT/GB2006/003719
(87) International publication number: WO 2007/039753

(56) References cited:
- WO-A1-98/45425
- WO-A2-02/29030
- FR-A- 2 477 572
- JIMENEZ J ET AL: "SELECTION OF ETHANOL-TOLERANT YEAST HYBRIDS IN PH-REGULATED CONTINUOUS CULTURE" APPLIED AND ENVIRONMENTAL MICROBIOLOGY, WASHINGTON,DC, US, vol. 54, no. 4, April 1988 (1988-04), pages 917-922, XP002072354 ISSN: 0099-2240
- HARTLEY B S ET AL: "DEVELOPMENT AND ECONOMICS OF A NOVEL THERMOPHILIC ETHANOL FERMENTATION" PRESENTATIONS FROM BIOTECH'83 LONDON 4-6 MAY, 1983 FIRST WORLD CONFERENCE, BIOTECH, NORTHWOOD,ONLINE CONF. LTD, GB, May 1983 (1983-05), pages 895-905, XP000999813
- PAYTON M A: "PRODUCTION OF ETHANOL BY THERMOPHILIC BACTERIA" TRENDS IN BIOTECHNOLOGY, ELSEVIER, AMSTERDAM, NL, vol. 2, no. 6, 1984, pages 153-158, XP000999007 ISSN: 0167-7799

## Description

### Field of the Invention

This invention relates to the production of microorganisms suitable for the production of ethanol as a product of bacterial fermentation.

### Background to the Invention

Bacterial metabolism can occurthrough various different mechanisms depending on the bacterial species and environmental conditions. Heterotrophic bacteria, which include all pathogens, obtain energy from oxidation of organic compounds, with carbohydrates (particularly glucose), lipids and protein being the most commonly oxidised compounds. Biological oxidation of these organic compounds by bacteria results in synthesis of ATP as the chemical energy source. The process also permits generation of more simple organic compounds (precursor molecules) which are required by the bacterial cell for biosynthetic reactions. The general process by which bacteria metabolise suitable substrates is glycolysis, which is a sequence of reactions that converts glucose into pyruvate with the generation of ATP. The fate of pyruvate in the generation of metabolic energy varies depending on the microorganism and the environmental conditions. There are three principle reactions of pyruvate.

First, under aerobic conditions, many micro-organisms will generate energy using the citric acid cycle and the conversion of pyruvate into acetyl coenzyme A, catalysed by pyruvate dehydrogenase (PDH).

Second, under anaerobic conditions, certain ethanologenic organisms can carry out alcoholic fermentation by the decarboxylation of pyruvate into acetaldehyde, catalysed by pyruvate decarboxylase (PDC) and the subsequent reduction of acetaldehyde into ethanol by NADH, catalysed by alcohol dehydrogenase (ADH).

A third process is the conversion of pyruvate into lactate which occurs through catalysis by lactate dehydrogenase (LDH).

There has been much interest in using micro-organisms for the production of ethanol using either micro-organisms that undergo anaerobic fermentation naturally or through the use of recombinant micro-organisms which incorporate genes involved in the production of ethanol. Although there has been some success in producing ethanol by using these micro-organisms, fermentation is often compromised by the increased concentration of the ethanol, especially where the micro-organism has a low level of ethanol tolerance.

Thermophilic bacteria have been proposed for ethanol production, and their use has the advantage that fermentation can be carried out at elevated temperatures which allows the ethanol produced to be removed as vapour at temperatures above 50°C; this also permits fermentation to be carried out using high sugar concentrations. However, finding suitable thermophilic bacteria which can produce ethanol efficiently is problematic.

WO01/49865 discloses a Gram-positive bacterium which has been transformed with a heterologous gene encoding pyruvate decarboxylase and which has native alcohol dehydrogenase function, for the production of ethanol. The bacterium is a thermophilic *Bacillus* and the bacterium may be modified by the inactivation of the lactate dehydrogenase gene using transposon insertion. The bacteria disclosed in WO01/49865 are all derived from *Bacillus* Strain LLD-R, a sporulation-deficient strain that arose spontaneously from culture, and in which the ldh gene has been inactivated by spontaneous mutation or by chemical mutagenesis. Strains LN and TN are disclosed as improved derivatives of strain LLD-R. However, all strains contain a Hae III type restriction systems that impedes plasmid transformation and therefore prevents the transformation within un-methylated DNA.

WO01/85966 discloses microorganisms that are prepared by *in vivo* methylation to overcome the restriction problems. This requires transformation with Hae III methyltransferase from *Haemophilus aegyptius* into strains LLD-R, LN and TN. However, strains LLD-R, LN and TN are unstable mutants and spontaneously revert to lactate-producing wild-type strains, particularly at low pH and in high sugar concentrations. This results in fermentation product changes from ethanol to lactate, making the strains unsuitable for ethanol production.

WO02/29030 discloses that strain LLD-R and its derivatives include a naturally-occurring insertion element (IE) in the coding region of the ldh gene. Transposition of this into (and out of) the ldh gene and subsequent gene inactivation is unstable, resulting in reversion. The proposed solution to this was to integrate plasmid DNA into the IE sequence.

WO98/45425 discloses a selection process to identify mutants of *Escherichia coli* KO11 that can grow and survive in high ethanol concentrations and are potentially useful for ethanol production.

Therefore, in the art, the production of microorganisms for ethanol production relies on modifying laboratory-produced chemically mutated *Bacillus* microorganisms, treating these with *in vivo* methylation procedures and further modifying the microorganisms to integrate plasmid DNA into the IE sequence. The procedure is complex, uncertain and there are also regulatory issues on how the strains can be used.

There is therefore a need for improved microorganisms for ethanol production.

### Summary of the Invention

According to a first aspect of the present invention, a method for the production of thermophilic microorganisms suitable for the production of ethanol comprises:
(i) culturing a thermophilic microorganism under aerobic or anaerobic conditions in a suitable culture media; and
(ii) incorporating increasing amounts of ethanol into the culture media to induce ethanol tolerance, wherein the wild-type thermophilic microorganism is modified by inactivation of the native lactate dehydrogenase gene, and wherein the native lactate dehydrogenase gene, or a portion thereof, has been deleted, and wherein the microorganism does not comprise a restriction system.

### Description of the Invention

The present invention is based on a treatment of a thermophilic microorganism to make the microorganism more tolerant to ethanol, and therefore, better able to produce ethanol. Increasing the ethanol tolerance of the microorganisms allows the microorganisms to be more resistant to the ethanol produced during their fermentation. This allows improvement in fermentation.

The method for the production of the thermophilic microorganisms involves culturing the thermophilic microorganisms under aerobic or anaerobic conditions in a suitable culture media and incorporating amounts of ethanol into the culture media to induce ethanol tolerance. In one embodiment increasing the ethanol into the culture media is carried out over time and usually in increments, to allow the microorganisms to adapt to the increased ethanol in the media. It is preferable to incorporate ethanol up to a final concentration of 3% w/v and then increase the ethanol concentration by 0.5% w/v, or less, until the concentration of ethanol in the media is at least 6% w/v more preferably, at least 7.5% w/v. In one embodiment, the initial culture media comprises 3% w/v ethanol and this is then increased by 0.5% increments to 6% w/v and then by 0.25% increments to 7.5% w/v.

During this procedure, the cell density can be monitored to ensure that cell growth is continuing. Preferably, if the cell density (determined by OD 600nm) falls by more than 25% and continues to decline as the ethanol concentration is increased, then the concentration of ethanol is allowed to fall to the previous highest level and the culture reestablished before continuing with the ethanol treatment.

An alternative method for the production of thermophilic organisms with higher ethanol tolerance involves continuous culturing the thermophilic microorganisms under aerobic or anaerobic conditions in appropriate culture medium. Once the culture has reached a steady state, where the growth of the organism has reached a constant rate (as determined by OD 600nm), an amount of ethanol is added, in one addition, to the culture in order to bring the ethanol to a specific desired concentration, for example 10 or 20% w/v of the set working volume of the culture. The continuous culture is continued at a low dilution rate, preferably 0.08-0.15 h⁻¹, allowing slow reduction of the ethanol concentration, until the original growth rate of the thermophilic organism is restored (as determine by OD 600nm). At this point a second amount of ethanol is added, the same quantity as the first and in one addition, to the culture, again in order to bring the ethanol to a specific desired concentration. The process of allowing the culture to recover to the original growth rate is repeated and further batches of ethanol are added until the culture is found to recover quickly, this being taken as less than twenty-four hours. At this point one of two outcomes will occur. Either ethanol tolerant strains are selected from this culture by sub-culturing at the desired ethanol concentration, preferably over 7.5% w/v, or larger quantities of ethanol are added to the culture and the process ethanol addition followed by growth rate recovery is repeated.

The microorganisms may be grown in defined media at 55°C - 65°C with a carbon limited substrate and a pH of 6.0 to 7.5 (preferably 6.3 to 7.2) at dilution rates of 0.08 to 0.5.

In batch culture, a process similar to the process described above can be performed, with growth in any suitable media with excess carbon and ethanol being added in early log phase growth. Cells from this initial culture can then be used at the end of the log phase growth to inoculate a fresh flask with an incremental amount of ethanol being added again at early log phase. This procedure may be repeated with incremental amounts of ethanol being added to the culture media at early log phase.

The thermophilic microorganisms to be used in the present invention are modified to delete the native lactate dehydrogenase gene, or a portion thereof.

This results in pyruvate metabolism being channelled away from lactate production and towards ethanol production, with enhanced levels of ethanol observed in lactate-negative mutants.

Inactivating the lactate dehydrogenase gene by detection of the gene helps to prevent the breakdown of pyruvate into lactate, and therefore promotes (under appropriate conditions) the breakdown of pyruvate into ethanol using pyruvate decarboxylase and alcohol dehydrogenase.

The wild-type microorganism may be any thermophilic microorganism, but it is preferred if the microorganism is of the *Bacillus spp.* In particular, it is preferred if the microorganism is of the *Geobacillus* species, in particular *Geobacillus thermoglucosidasius.*

The microorganisms may be "wild-type", i.e. they are not laboratory-produced mutants. The microorganisms may be isolated from environmental samples expected to contain thermophiles. Isolated wild-type microorganisms will have the ability to produce ethanol but, unmodified, lactate is likely to be the major fermentation product. The isolates are also selected for their ability to grow on hexose and/or pentose sugars at thermophilic temperatures.

II Furthermore, the microorganism has no restriction system, thereby avoiding the need for *in vivo* methylation. It is preferable that the microorganism of the invention has certain desirable characteristics which permit the microorganism to be used in a fermentation process. The microorganism should have the ability to utilise C5 and C6 sugars as a substrate, including cellubiose and starch. It is preferable if the microorganism is transformable at a high frequency. Furthermore, the microorganism should have a growth rate in continuous culture of above 0,3hr⁻¹,

The microorganism will be a thermophile and will grow in the temperature range of 40°C - 85°C. Preferably, the microorganism will grow within the temperature range 50°C-70°C. In addition, it is desirable that the microorganism grows in conditions of pH 6.5 or below, in particular pH6.5-pH4.5.

The nucleic acid sequence for lactate dehydrogenase is now known. Using this sequence, it is possible for the skilled person to target the lactate dehydrogenase gene to achieve deletion of the gene sequence, or a portion of the gene sequence. Deletion avoids the difficulty of reactivation of the gene sequence which is often experienced when transposon inactivation is used.

In a preferred embodiment, the micro-organism comprises a heterologous alcohol dehydrogenase gene and a heterologous pyruvate decarboxylase gene. The expression of these heterologous genes results in the production of enzymes which redirect the metabolism so that ethanol is the primary fermentation product. These genes may be obtained from micro-organisms that typically undergo an aerobic fermentation, including *zymomonas* species, including *zymomonas mobilis.*

Methods for the preparation and incorporation of these genes into microorganisms are known, for example in Ingram et al, Biotech & BioEng, 1998; 58 (2+3): 204-214 and US 5916787. The genes may be introduced in a plasmid or integrated into the chromosome, as will be appreciated by the skilled person.

The microorganisms of the invention may be cultured under conventional culture conditions, depending on the thermophilic microorganism chosen. The choice of substrates, temperature, pH and other growth conditions can be selected based on known culture requirements, for example see WO01/49865 and WO01/85966. Suitable culture and fermentation conditions are indicated in Tables 1, 2 and 3:

**Table 1**

| **Chemical** | **Vol./L** | **Final Conc.** |
|---|---|---|
| NaH₂PO₄.2H₂O | | 25 mM |
| K₂SO₄ | | 10 mM |
| Citric acid. H₂O | | 2 mM |
| MgSO₄.7H₂O | | 1.25 mM |
| CaCl₂.2H₂O | | 0.02 mM |
| Sulphate TE Solution | 5 ml | See below |
| Na₂MoO₄.2H₂O | | 1.65 µM |
| **Yeast Extract** | **10g** | |
| Antifoam | 0.5ml | |
| | | |

| **Post-auto addns :** | | |
|---|---|---|
| 4M Urea | 25 ml | 100 mM |
| 1% Biotin | 300 µl | 12 µM |
| | | |
| 20% Glucose² | 50ml | 1% |

**Table 2**

| **Sulphate Trace Elements Stock Solution** | | | |
|---|---|---|---|
| **Chemical** | **gl⁻¹ (ml)** | **gl⁻¹ (ml)** | **Medium Conc.** |
| Conc. H₂SO₄ | 5 ml | 50 ml | |
| ZnSO₄.7H₂O | 1.44 | 14.4 | 25 µM |
| FeSO₄.7H₂O | 5.56 | 55.6 | 100 µM |
| MnSO₄.H₂O | 1.69 | 16.9 | 50 µM |
| CuSO₄.5H₂O | 0.25 | 2.5 | 5 µM |
| CoSO₄.7H₂O | 0.562 | 5.62 | 10 µM |
| Ni SO₄.6H₂O | 0.886 | 8.86 | 16.85 µM |
| H₃BO₃ | 0.08 | | |
| Del- H₂O (final | 1000 ml | 10 litres | |

**Table 3**

| **Fermenter Conditions** | |
|---|---|
| | |
| Inoculum | 10% v/v |
| Volume | 1000 ml |
| Temperature | 60°C |
| PH | 7.0 controlled with NaOH |
| Aeration | 0.4 vvm |
| N₂ flow | 0.05 lpm |
| Aqitation | 400 rpm |
| Media | Urea Sulphates CDM for Fermenters |
| Sugar feed | 100 ml 50% glucose |
| Antifoam | |

The invention is illustrated in the following Example, with reference to the accompanying drawings.

### Example

The following media were prepared:
SAM2 - perL

| | |
|---|---|
| Yeast extract | 1.0g |
| Tryptone | 0.5g |
| NH₄Cl | 1.0g |
| NaH₂PO₄ | 0.5g |
| MgSO₄.7H₂O | 0.2g |
| KCI | 0.2g |
| MnCl₂.4H₂O | 3mg (add 100µL of a 30mg/mL stock) |
| CaCl₂.2H₂O | 5mg (add 100µL of a 50mg/mL stock) |
| PIPES buffer | 12.096g |

Total volume in distilled water: 950mL, adjust to pH 7.0 with NaOH or H₂S0₄. Autoclave.
After cooling 2.5mL of Sulphates Trace Elements stock solution was added (see Table 2), together with 50mL 20% w/v filter sterilized sugar solution.
Modified US (Urea Salts) Media (USM)

| | |
|---|---|
| Glucose | 10.0 g/L |
| Yeast extract | 0.8 g/L |
| Citric acid | 0.42 g/L |
| MgSO₄ | 0.31 g/L |
| NaH₂PO₄ | 3.1 g/L |
| K₂SO₄ | 3.5 g/L |
| Urea | 3.0 g/L |
| CaCl₂ | 2 mg/L |
| Na₂MoO₄ | 4 mg/L |
| Trace element solution (Table 2) | 5.0 ml/L |

For solid media 20.0 g/L of bacto-agar was added.
Corrected to pH 7 prior to sterilisation with 3M NaOH
TGP medium

| | |
|---|---|
| Bacto tryptone | 17.0 g/L |
| Soy peptone | 3.0 g/L |
| NaCl | 5.0 g/L |
| K₂HPO₄ | 2.5 g/L |
| Sodium pyruvate | 4.0 g/L |
| Glycerol | 4.0 mL/L |

For solid media 20.0 g/L of bacto-agar was added. The medium was corrected to pH 7 prior to sterilisation with 3M NaOH.

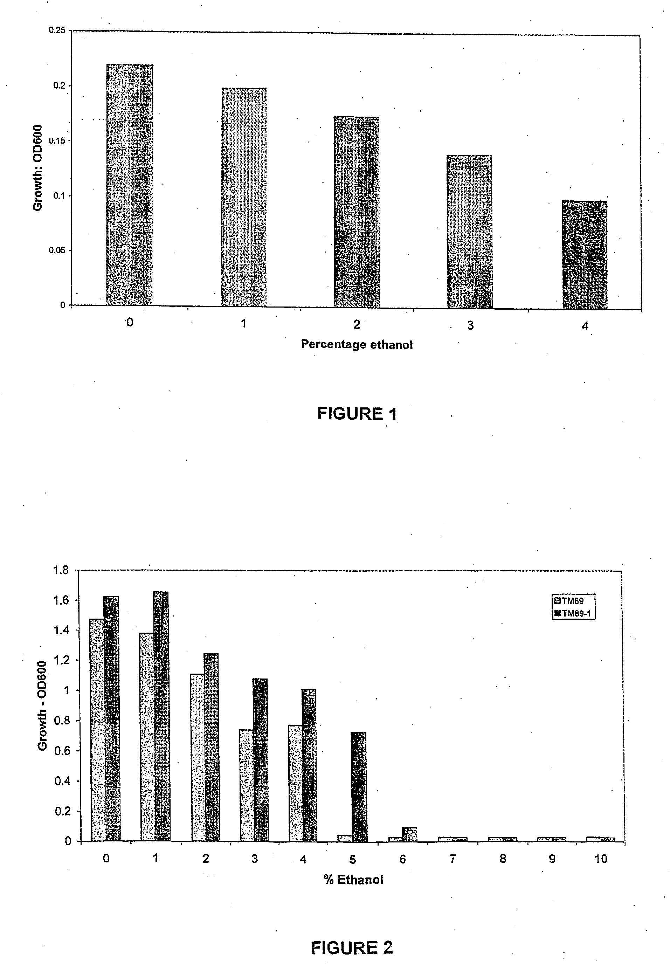

Ethanol tolerance of a wild-type organism (NCIMB 11955) was tested in order to determine the starting point. The organism was grown overnight (LB agar plate, 60°C) and a colony used to inoculate an overnight culture (100mL USM, 1% glucose, 60°C, 250rpm). This culture was then used to inoculate a triplicate series of flasks containing 0, 1, 2, 3, or 4% ethanol which were then grown for 36 hours before the growth was measured (50mL USM, 1% glucose, 60°C, 250rpm). The results are shown in Figure 1 and suggest that NCIMB 11955 will not tolerate more than 4% ethanol.

Using this result as a basis for comparison, experiments were performed to increase the ethanol tolerance of mutant TM89 to 8% v/v ethanol.

### Fermentation Method

| | |
|---|---|
| Microorganism: | Gt TM-89 |
| Inoculum: | 50ml 2xYT culture (7% v/v) |
| Apparatus: | LH glass fermenter (700ml working volume) |
| | Temperature and pH control with the Anglicon control system |
| | Mixed with a magnetic stirrer. |
| Set values: | Temperature: 60°C |
| | pH: 6.80 |
| | air: 0.2-0.4vvm |
| | stirrer speed: 225rpm |
| | flow rate controlled with Watson Marlow Pump (0-100 ml/hr) |
| Medium: | SAM2 2% glucose, 0.05% organic antifoam (see media sheet). |
| | PH controlled using 10% NaOH |
| Ethanol spike: | 75ml or 150ml ethanol (10-20% spike) |

The strategy adopted was to:
1) Achieve steady states and measure product yields/sugar utilization; and
2) Spike culture with ethanol, allow culture to recover (ethanol will wash out over time), remove samples and prepare glycerol stocks, repeating step (2) as necessary.

In order to evaluate ethanol tolerance, the following protocol was developed.
1. Add 5ml of TGP broth to two sterile universals.
2. Add 100µl of TM-89 and TM89-1 glycerol stock to each tube respectively.
3. Culture for 5-6 hours (therefore active cells, as they should be in log phase) at 60°C with shaking.
4. Measure the OD₆₀₀ of each broth (so you know the starting of OD₆₀₀).
5. Prepare 11 sterile universal tubes with a final volume of 10ml TGP broth containing 0%, 1%, 2%, 3%, 4%, 5%, 6%, 7%, 8%, 9% 10% (v/v) concentrations of ethanol, in duplicate for each TM-89 strain (ie. 44 tubes).
6. Inoculate each tube with 100µl of cells from the corresponding 5ml TGP broths from stages 1 + 2.
7. Culture overnight at 60°C with shaking.
8. Remove 1ml from each tube, dilute 1:5 and measure the OD⁶⁰⁰ against H₂O blank.

The results were analysed as follows:
(1) Optical density of culture measured using a Jencons Spectrophotometer [cell concentration (g/L) was calculated from A₆₀₀ x 0.3].
(2) Glucose concentration was measured using a blood glucose meter (Roche).
(3) Ethanol concentration measured by using an enzyme based assay kit (supplied by R-Biopharm).

The results are illustrated in Figure 2. The strain isolated at the end of the fermentation displayed consistently higher OD values than the starting TM89 strain in TGP and in TGP with a range of ethanol concentrations. There was a significant difference in growth at 5% ethanol indicating improved ethanol tolerance.

## Claims

1. A method for the production of thermophilic microorganisms suitable for the production of ethanol, comprising:
i. culturing a thermophilic microorganism under aerobic or anaerobic conditions in a suitable culture media; and
ii. incorporating amounts of ethanol into the culture media to induce ethanol tolerance;
wherein the wild-type thermophilic microorganism is modified by inactivation of the native lactate dehydrogenase gene, and wherein the native lactate dehydrogenase gene, or a portion thereof, has been deleted, and wherein the microorganism does not comprise a restriction system.

2. A method according to claim 1, wherein an increasing amount of ethanol is added into the culture media, with the microorganisms allowed to adapt to the added ethanol prior to the next incremental addition of ethanol.

3. A method according to claim 1 or claim 2, wherein the ethanol is incorporated to a final concentration of at least 3% w/v.

4. A method according to any preceding claim, wherein the ethanol is incorporated to a final concentration of at least 6% w/v.

5. A method according to any preceding claim, wherein the ethanol is incorporated to a final concentration of at least 7.5% w/v.

6. A method according to any preceding claim, wherein the ethanol is incorporated in increments of 0.5% w/v or less.

7. A method according to any preceding claim, wherein the ethanol is incorporated to the culture media at early log phase.

8. A method according to any preceding claim, wherein the microorganism is *Geobacillus thermoglucosidasius.*

9. A method according to any preceding claim, wherein the microorganism comprises a heterologous pdc gene.

10. A method according to any preceding claim, wherein the microorganism comprises a heterologous *adh* gene.

11. A method according to any preceding claim, wherein the microorganism can metabolise cellobiose and/or starch.

12. A method according to any preceding claim, wherein the microorganism is transformable at high frequency.

13. A method according to any preceding claim, wherein the microorganism grows at a temperature from 40°C - 85°C, preferably 50° - 70°C.

## Patentansprüche

1. Verfahren zur Herstellung thermophiler Mikroorganismen, die für die Ethanolherstellung geeignet sind, umfassend:
i. Kultivierung eines thermophilen Mikroorganismus unter aeroben oder anaeroben Bedingungen in einem geeigneten Kulturmedium; und
ii. Einbringung von Ethanolmengen in das Kulturmedium zwecks Bewirkung von Ethanoltoleranz;
wobei der thermophile Mikroorganismus vom Wildtyp durch Inaktivierung des nativen Laktatdehydrogenase-Gens modifiziert ist und wobei das native Laktatdehydrogenase-Gen oder ein Teil desselben deletiert wurde und wobei der Mikroorganismus kein Restriktionssystem umfasst.

2. Verfahren nach Anspruch 1, wobei dem Kulturmedium eine zunehmende Menge Ethanol zugegeben wird, wobei man die Mikroorganismen vor dem jeweils nächsten Ethanolzugabeschritt sich an das zugegebene Ethanol gewöhnen lässt.

3. Verfahren nach Anspruch 1 oder Anspruch 2, wobei das Ethanol bis zu einer Endkonzentration von mindestens 3 Gew./Vol.-% eingebracht wird.

4. Verfahren nach einem vorangehenden Anspruch, wobei das Ethanol bis zu einer Endkonzentration von mindestens 6 Gew./Vol.-% eingebracht wird.

5. Verfahren nach einem vorangehenden Anspruch, wobei das Ethanol bis zu einer Endkonzentration von mindestens 7,5 Gew./Vol.% eingebracht wird.

6. Verfahren nach einem vorangehenden Anspruch, wobei das Ethanol in Schritten von 0,5 Gew./Vol.-% oder weniger eingebracht wird.

7. Verfahren nach einem vorangehenden Anspruch, wobei das Ethanol in der frühen log-Phase in das Kulturmedium eingebracht wird.

8. Verfahren nach einem vorangehenden Anspruch, wobei der Mikroorganismus *Geobacillus thermoglucosidasius* ist.

9. Verfahren nach einem vorangehenden Anspruch, wobei der Mikroorganismus ein heterologes *pdc-Gen* umfasst.

10. Verfahren nach einem vorangehenden Anspruch, wobei der Mikroorganismus ein heterologes adh-Gen umfasst.

11. Verfahren nach einem vorangehenden Anspruch, wobei der Mikroorganismus Cellobiose und/oder Stärke verstoffwechseln kann.

12. Verfahren nach einem vorangehenden Anspruch, wobei der Mikroorganismus bei hoher Frequenz transformierbar ist.

13. Verfahren nach einem vorangehenden Anspruch, wobei der Mikroorganismus bei einer Temperatur von 40 °C - 85 °C, bevorzugt 50 °C - 70 °C, wächst.

## Revendications

1. Procédé pour la production de micro-organismes thermophiles approprié pour la production d'éthanol, comprenant :
i. la culture d'un micro-organisme thermophile dans des conditions aérobies ou anaérobies dans un milieu de culture approprié ; et
ii. l'incorporation de quantités d'éthanol dans le milieu de culture pour induire une tolérance à l'éthanol ;
dans lequel le micro-organisme thermophile de type sauvage est modifié par l'inactivation du gène natif de la lactodéshydrogénase, et dans lequel le gène natif de la lactodéshydrogénase, ou une portion de celui-ci, a été délété, et dans lequel le micro-organisme ne comprend pas de système de restriction.

2. Procédé selon la revendication 1, dans lequel une quantité croissante d'éthanol est ajoutée dans le milieu de culture, les micro-organismes ayant la possibilité de s'adapter à l'éthanol ajouté avant l'ajout incrémentiel suivant d'éthanol.

3. Procédé selon la revendication 1 ou la revendication 2, dans lequel l'éthanol est incorporé à une concentration finale d'au moins 3 % p/v.

4. Procédé selon l'une quelconque des revendications précédentes, dans lequel l'éthanol est incorporé à une concentration finale d'au moins 6 % p/v.

5. Procédé selon l'une quelconque des revendications précédentes, dans lequel l'éthanol est incorporé à une concentration finale d'au moins 7,5 % p/v.

6. Procédé selon l'une quelconque des revendications précédentes, dans lequel l'éthanol est incorporé à des incréments de 0,5 % p/v ou moins.

7. Procédé selon l'une quelconque des revendications précédentes, dans lequel l'éthanol est incorporé au milieu de culture lors de la phase log précoce.

8. Procédé selon l'une quelconque des revendications précédentes, dans lequel le micro-organisme est le *Geobacillus thermoglucosidasius.*

9. Procédé selon l'une quelconque des revendications précédentes, dans lequel le micro-organisme comprend un gène pdc hétérologue.

10. Procédé selon l'une quelconque des revendications précédentes, dans lequel le micro-organisme comprend un gène *adh* hétérologue.

11. Procédé selon l'une quelconque des revendications précédentes, dans lequel le micro-organisme peut métaboliser le cellobiose et/ou l'amidon.

12. Procédé selon l'une quelconque des revendications précédentes, dans lequel le micro-organisme est transformable à haute fréquence.

13. Procédé selon l'une quelconque des revendications précédentes, dans lequel le micro-organisme croît à une température de 40 °C à 85 °C, de préférence 50 °C à 70 °C.
